# EUROPEAN PATENT SPECIFICATION

(11) **EP 0 392 626 B1**
(45) Date of publication and mention of the grant of the patent: **14.12.1994**
(21) Application number: 90200865.5
(22) Date of filing: 10.04.1990
(51) Int. Cl.: A61N 2/04

(54) **Apparatus for magnetic applications by means of pulsating magnetic fields, in particular for magnetotherapy**
Apparat für magnetische Anwendungen mittels pulsierender magnetischer Felder, insbesondere für Magnetotherapie
Appareil pour applications magnétiques au moyen de champs magnétiques pulsés, en particulier pour magnétothérapie

(30) Priority: 14.04.1989 IT 2014589
(43) Date of publication of application: 17.10.1990
(73) Proprietor: Caprotti, Guido, Hyde Park Square, London (GB)
(72) Inventor: Ceccarani, Franco, I-20122 Milan (IT)
(74) Representative: Fusina, Gerolamo

(56) References cited:
- EP-A- 0 244 784
- DE-U- 8 714 410
- FR-A- 716 954

## Description

The present invention relates to an apparatus for magnetic applications by means of pulsating magnetic fields, in particular for magnetotherapy.

The beneficial action exerted by the application of magnetic fields of suitable intensity, frequency and polarity to parts of the human body has been long known, and in this regard proper apparatuses have also been proposed in the past, which are substantially composed by a kind of parallelepipedal box, with a horizontal top plane capable of receiving, laid down, the body of a patient. The plane has a, length equivalent to the length of the box, and a smaller width, so as to leave longitudinal slots open alongside said box.

Around the plane a substantially annular element is positioned, with its axis being parallel to the longitudinal direction of the plane. Said annular element is provided with windings for the generation of a magnetic field, the force lines of which run through said annular element in the axial direction, and close upon themselves outside said annular element.

The annular element is capable of being reciprocated along the box in the longitudinal direction of the plane, so that the patient, laid down on the plane in an extended position through the annular element, is submitted to a progressive treatment of the various parts of his body by the magnetic field generated by the annular element, or he can be submitted to the treatment of pre-established parts of his body, by means of a suitable positioning of the annular element along the plane.

One can easily understand that such an apparatus, besides being very cumbersome, is very complex owing to the motion control mechanisms which must enable the annular element to reciprocate together with its windings.

Furthermore, this kind of apparatus allows only a portion of a patient's body to be treated at a same time, i.e., that part of the patients body where the magnetic-field-generating annular element is at that time, whilst the other parts of the patient's body can be treated at a later time only, after the annular element has been moved to a position adjacent these parts.

Further apparatus for magnetic applications are known from DE-U-8 714 410 and FR-A-716 954.

The apparatus of DE-U-8 714 410, which has the features of the preamble of claim 1, comprises a container body in the form of a cushion and the windings are all connected in series or in parallel and are all fed simultaneously by a same current supply through a pulse generator. Such an apparatus is cheap to be manufactured and simple to be used, but it is suitable only for local applications on a specific sore region of the human body.

The apparatus of FR-A-716 954 comprises serially connected pairs of windings within a container body which can take the form of a mattress. The windings of each pair are of different nature for respectively providing thermic and electromagnetic effects and a timer is provided to temporarily interrupt the current supplied simultaneously to all the windings when the heating effect of the windings reaches a predetermined value. Apart from the fact that this document does not suggest to supply the windings with pulsating currents, the apparatus known from this document only allows to uniformly treat the whole human body.

A further drawback common to both the apparatus of the cited documents lies in the fact that movements of the patient relative to the cushion or the mattress may change the position of the windings and so the force lines of the magnetic fields may assume different directions with respect to those required for a beneficial treatment of a specific part of the patient's body.

In view of these drawbacks and limitations of the apparatus as known from the prior art, the problem arises of providing an apparatus which is capable of overcoming them, and precisely a purpose of the instant invention is that of providing a structure for magnetic applications by means of pulsating magnetic fields, in particular for magnetotherapy, which is more handy and efficacious, in the meaning that it is simpler and is capable of simultaneously treating large portions of a human body, in particular also the whole patient's body, but also makes it possible specifically different or localized treatments to be carried out without the equipment having to undergo any particular modifications, and which can be used for out-patient treatments or for hospital treatments, as well as for performing treatments at patient's home.

Not least purpose of the invention is that of providing an economically favourable and easily transportable equipment.

These and still further purposes are achieved by an apparatus for magnetic applications by means of pulsating magnetic fields, in particular for magnetotherapy, having the features of claim 1.

Advantageously, with an apparatus of such a kind, the treatment of the patient can be carried out simultaneously at each point of the treatment plane, and a structure movable along the body of the patient is no longer needed. A simpler and cheaper apparatus is the result. The distributed arrangement of the windings mates it possible a more homogeneous magnetic field to be obtained through the whole treatment region, with benecial effects for the patient.

The various windings can be regulated either individually or as groups, so as to generate magnetic fields whose intensities and frequencies are different according to the treatment regions or treatment requirements They can also be installed adjustable in position relatively to the plane of treatment. The carrier structure which supports the windings can be connected with a separate generator apparatus, capable of supplying the currents for producing the desired intensities of the magnetic field generated by each one of the various windings. Thanks to this feature, a more versatile and efficacious apparatus is obtained.

Further details and advantages of the invention will be clearer from the following disclosure of some exemplifying forms of practical embodiment of the same invention, as illustrated for non-limitative purposes in the hereto attached drawings, in which:
Figure 1 shows a perspective view of an apparatus according to the present invention, partially opened for the purposes of a better understanding;
Figure 2 shows a schematic view of a possible layout of the windings and of the relevant electrical connections with the generator apparatus;
Figure 3 shows a perspective view of a winding used in the apparatus according to the instant invention;
Figure 4 shows a perspective view of a second examplifying form of practical embodiment of an apparatus according to the instant invention;
Figure 5 schematically shows, on a smaller scale than that of the preceding Figures, a further form of practical embodiment of an apparatus according to the invention.

Referring to the drawings, an apparatus for magnetic applications by means of pulsating magnetic fields, in particular for magnetotherapy, comprises a treatment structure 1 essentially constituted by a treatment plane 2 defined by a container body 3, made integral with each Other with the aid of means not shown in detail. The structure 1 preferably has the shape of a parallelepipedal panel, whose top major face defines the treatment plane 2 which extends for receiving a laid down human body. Under the treatment plane 2, inside the interior of the container body 3, which has a rigid structure, magnetic elements are installed, which generate magnetic fields at the treatment plane 2. Such magnetic elements are constituted by a plurality of windings 4 of substantially annular shape, positioned spaced apart from one another and with their axes being substantially perpendicular to the surface of the treatment plane 2.

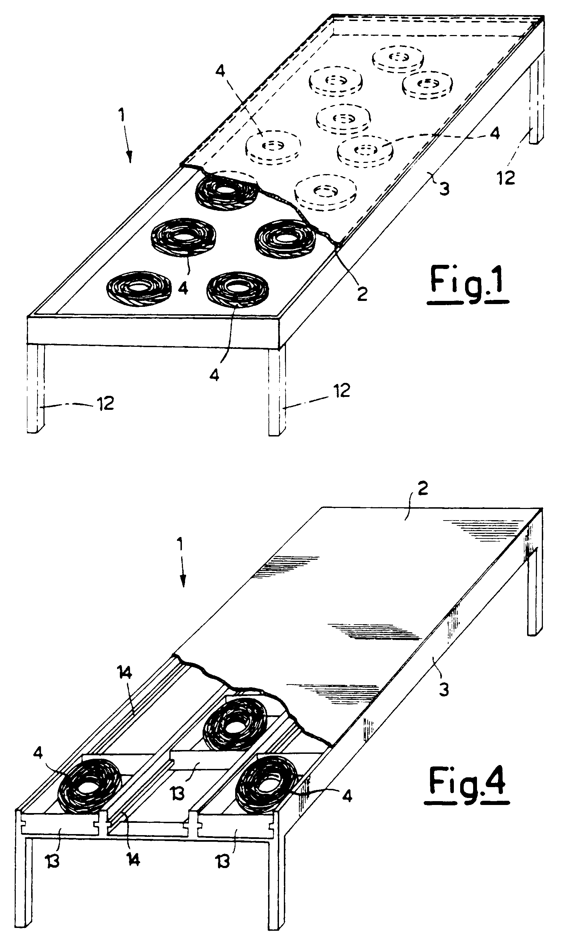

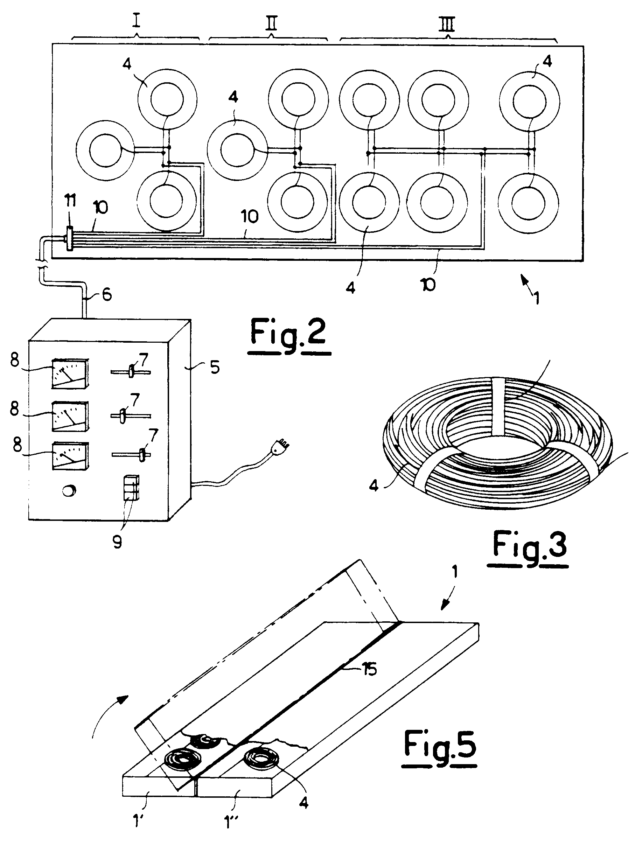

The windings 4 are windings in air, i.e., they are not wound on iron cores, and preferably have a flattened, substantially ring shape, as one can better see in Figure 3. They are preferably installed fixed inside the container body 3 With different densities according to a distribution pattern depending on the treatment needs. For example, as one can see in Figures 1 and 2, they can be positioned partially side-by-side as symmetrical pairs relatively to the longitudinal plane of symmetry of the patient treatment plane 2, and partially as single windings arranged centered relative to said longitudinal plane of symmetry, with the first single winding 4 corresponding to the position of the head of the patient laid down on the treatment plane 2, and the other single windings corresponding to the positions of the various parts of patient's body. The layout of the windings 4 is furthermore such as to create a respective magnetic field whose north polarity is directed towards the treatment plane 2.

The windings 4 are connectable, either as individual windings, or as groups of windings, to respective electrical sources capable of generating inside said windings pulsating currents which can be regulated in intensity and in frequency. As one can see, e.g., in Figure 2, the windings 4 can be electrically connected with one another in such a way as to form electrically separate groups I, II, III, with each group including at least one winding 4 and being capable of being independently connected with a respective electrical source, which can be installed in a generator equipment separate from the structure 1, like the one indicated with the reference numeral 5, connected with the structure 1 by means of a cable 6. On the front panel-board of the generator equipment 5 the knobs 7 of regulation of the currents fed to the windings 4, or to the groups of windings 4, are installed together with the respective indicator instruments 8 which display the current intensity values. The reference numeral 9 indicates pushbuttons which sake it possible to exclude particular individual windings 4 or groups of windings 4 so as to cause the windings 4 or groups of windings 4 to be operable selectively and independently by the respective electrical supply sources.

Advantageously, the wires 10 of the various windings 4 inside the structure 1 can be connected with the terminals of a terminal box 11 installed at an end of the structure 1. The cable 6 coming from the generator equipment 5 is connected with said terminal box 11 as well.

The structure 1 is advantageously entirely made from an amagnetic material. It can be provided with support legs 12, so as to practically constitute a kind of a bed for the treatment of the patient, or it can be placed upon a table, and fastened to this latter. The same structure 1, thanks to its dimensions, can be easily placed on existing beds, or can be provided with a top mattress for a relaxing treatment of the patient.

The configuration of the windings 4 and their dimensions are so selected as to have a minimal difference in magnetic field intensity between the centre of the windings 4 and the outer edge, so as to have magnetic fields which are relatively homogeneous as to intensity and density. Outside the windings 4, the magnetic field advantageously has lines of force running a long a rather wide arc. The distances between the windings 4 are so selected as not to intersect the lines of force of each one of the other windings, and to obtain a relative homogeneity of field throughout the region concerned by the installation of the windings 4.

For the purposes of a magnetotherapeutical treatment, magnetic field intensities of, e.g., a few tens of Gauss generated by the windings 4, by means of pulsating, half-wave currents with values of, e.g., 200 mA, resulted advantageous. The treatment times can be variable , and can be preset in the generator 5 by means of suitable timers. The generator 5 can be equipped with electronic means with a pre-established treatment program, which could also include local or general treatment pause times.

For a particularly efficacious treatment, the windings 4 could be positioned from time to time adjacent the vital points of the patient's body and therefore the windings 4 could be made capable of being positioned at different places inside a same container body 3. An examplifying form of a structure with windings 4 adjustable in position is shown in Figure 4. One can see that the windings 4 are each positioned inside a substantially drawer-like container element 13, with said elements 13 being slidable along guides 14 extending in the longitudinal direction of the patient treatment plane 2 and of the container body 3. The elements 13 can be fastened at a pre-established position along the guides 14 with the aid of fastening means not shown in detail. In order to make it possible the elements 13 to be displaced, the container 3 is open at its bottom.

Another possible form of practical embodiment of an apparatus according to the present invention is depicted in Figure 5, wherein the structure 1 is formed by two half-structures 1' and 1'' of substantial parallelepipedal shape, and hinged to each other along a longitudinal edge 15 thereof, so as to create a pliable structure. One could also think of course of a structure capable of being folded along a transversal line.

One can understand from the above disclosure that an apparatus according to the present invention solves the purposes as initially declared and results to be an extremely versatile and efficacious one, with the possibility of the magnetic act ion on the various parts of the patient's body being varied on choice and to the most suitable extent. The apparatus has small overall dimensions and is cheap.

Of course, many different forms of practical embodiment are possible besides the hereinabove illustrated forms. For example, the electrical regulation and generation equipment could be integrated in the structure 1.

## Claims

1. Apparatus for magnetic applications by means of pulsating magnetic fields, in particular for magnetotherapy, comprising a container body (3) defining a treatment plane (2), and a plurality of windings (4) for generating magnetic fields at said treatment plane (2), said windings (4) being housed in said container body (3) spaced from one another and having a substantially annular shape with their axes substantially perpendicular to said treatment plane (2), said windings (4) being windings in air and being connectable with a supply source for generating a pulsating current in said windings (4), characterised in that said container body (3) is a rigid structure and said treatment plane (2) extends for receiving a laid down human body, and in that said windings (4) form electrically separate groups (I, II, III) each including at least one winding (4), said groups (I, II, III) being operable selectively and independently by respective electrical pulsating current supply sources.

2. Apparatus according to claim 1, characterised in that the electrical pulsating current supply sources are arranged in a generator equipment (5) separate from said container body (3).

3. Apparatus according to claim 1 or 2, characterised in that said pulsating current supply sources include means (7) for regulating the frequency and intensity of the pulsating current.

4. Apparatus according to one of claims 1 to 3, characterised in that said windings (4) are adjustable in position within said container body (3).

5. Apparatus according to claim 4, characterised in that said windings (4) are arranged inside container elements (13) slidable along guides (14) extending in the longitudinal direction of said container body (3).

6. Apparatus according to claim 5, characterised in that said container body (3) is open at its bottom for allowing displacement of said container elements (13).

7. Apparatus according to one of claims 1 to 6, characterised in that said container body (3) is provided with support legs (12).

8. Apparatus according to one of claims 1 to 7, characterised in that the windings (4) are arranged so that the north polarity of the magnetic field generated by the windings (4) is directed towards said treatment plane (2).

9. Apparatus according to one of claims 1 to 8, characterised in that said groups (I, II, III) include side-by-side windings (4) arranged pairwise symmetrically relative to the longitudinal plane of symmetry of said treatment plane (2), and single windings (4) arranged centered relative to said longitudinal plane of symmetry.

10. Apparatus according to one of claims 1 to 9, characterised in that said groups (I, II, III) comprise windings (4) arranged with different density within the container body (3).

## Patentansprüche

1. Vorrichtung für magnetische Anwendungen mittels pulsierender magnetischer Felder, insbesondere für Magnetotherapie, bestehend aus einem eine Behandlungsebene (2) definierenden Gehäuseteil (3) und einer Mehrzahl von Wicklungen (4) zur Erzeugung magnetischer Felder an der Behandlungsebene (2), wobei die Wicklungen (4) im Gehäuseteil (3) mit Abstand voneinander untergebracht sind und im wesentlichen Ringform mit ihren Achsen im wesentlichen senkrecht zur Behandlungsebene (2) besitzen, und die Wicklungen (4) Wicklungen mit Luftkern sind und mit einer Stromquelle zur Erzeugung eines pulsierenden Stroms in den Wicklungen (4) verbindbar sind, dadurch gekennzeichnet, daß das Gehäuseteil (3) eine starre Ausbildung hat und seine Behandlungsebene (2) eine Erstreckung zur Aufnahme eines niedergelegten menschlichen Körpers aufweist und daß die Wicklungen (4) elektrisch getrennte Gruppen (I,II,III) bilden, die jeweils zumindest eine Wicklung (4) enthalten, wobei die Gruppen (I,II,III) in wahlweiser und unabhängiger Form von jeweiligen Stromquellen für den pulsierenden elektrischen Strom betätigbar sind.

2. Vorrichtung nach Anspruch 1, dadurch gekennzeichnet, daß die Stromquellen für den pulsierenden elektrischen Strom in einer Generatoreinrichtung (5) getrennt vom Gehäuseteil (3) angeordnet sind.

3. Vorrichtung nach Anspruch 1 oder 2, dadurch gekennzeichnet, daß die Stromquellen für den pulsierenden Strom Einrichtungen (7) zum Regulieren der Frequenz und der Stärke des pulsierenden Stroms umfassen.

4. Vorrichtung nach einem der Ansprüche 1 bis 3, dadurch gekennzeichnet, daß die Wicklungen (4) in ihrer Position innerhalb des Gehäuseteils (3) einstellbar sind.

5. Vorrichtung nach Anspruch 4, dadurch gekennzeichnet, daß die Wicklungen (4) im Inneren von Gehäuseelementen (13) angeordnet sind, die entlang in Längsrichtung des Gehäuseteils (3) verlaufender Führungen (14) verschieblich sind.

6. Vorrichtung nach Anspruch 5, dadurch gekennzeichnet, daß das Gehäuseteil (3) an seinem Boden zur Ermöglichung einer Verschiebung der Gehäuseelemente (13) offen ist.

7. Vorrichtung nach einem der Ansprüche 1 bis 6, dadurch gekennzeichnet, daß das Gehäuseteil (3) mit Stützfüßen (12) versehen ist.

8. Vorrichtung nach einem der Ansprüche 1 bis 7, dadurch gekennzeichnet, daß die Wicklungen (4) so angeordnet sind, daß der Nordpol des von den Wicklungen (4) erzeugten magnetischen Feldes zu der Behandlungsebene (2) hin gerichtet ist.

9. Vorrichtung nach einem der Ansprüche 1 bis 8, dadurch gekennzeichnet, daµ die Gruppen (I,II,III) nebeneinanderliegende Wicklungen (4), die paarweise symmetrisch zur Symmetrielängsebene der Behandlungsebene (2) angeordnet sind, und einzelne Wicklungen (4) umfassen, die zentriert zur Symmetrielängsebene angeordnet sind.

10. Vorrichtung nach einem der Ansprüche 1 bis 9, dadurch gekennzeichnet, daß die Gruppen (I,II,III) Wicklungen (4) umfassen, die mit unterschiedlicher Dichte im Gehäuseteil (3) angeordnet sind.

## Revendications

1. Appareil pour applications magnétiques au moyen de champs magnétiques à impulsions, en particulier pour la magnétothérapie, comprenant un corps de conteneur (3) définissant un plan de traitement (2) et une pluralité d'enroulements (4) destinée à produire des champs magnétiques au niveau du dudit plan de traitement (2), lesdits enroulements (4) étant logés dans le dit corps de conteneur (3) espacés les uns des autres et ayant une forme essentiellement annulaire avec leurs axes substantiellement perpendiculaires au dit plan de traitement (2), lesdits enroulements (4) étant des enroulements à l'air libre et pouvant être reliés à une source d'alimentation destinée à générer un courant à impulsions dans lesdits enroulements (4), caractérisé en ce que ledit corps de conteneur (3) est une structure rigide et ledit plan de traitement (2) s'étend de manière à recevoir un corps humain allongé, et en ce que lesdits enroulements (4) forment des groupent électriquement séparés (I,II,III) comprenant chacun au moins un enroulement (4), lesdits groupes (I,II,III) pouvant être actionnés de manière sélective et indépendante par différentes sources respectives d'alimentation en courant à impulsions.

2. Appareil selon la revendication 1, caractérisé en ce que les sources d'alimentation en courant à impulsions sont placées dans un équipement générateur (5) séparé du dit corps de conteneur (3).

3. Appareil selon la revendication 1 ou 2, caractérisé en ce que lesdites sources d'alimentation en courant à impulsions comprennent des moyens (7) de régulation de la fréquence et de l'intensité du courant à impulsions.

4. Appareil selon l'une quelconque des revendications 1 à 3, caractérisé en ce que les enroulements (4) sont réglables du point de vue de leur position à l'intérieur du corps de conteneur (3).

5. Appareil selon la revendication 4, caractérisé en ce que lesdits enroulements (4) sont placés à l'intérieur d'éléments de conteneur (13) pouvant coulisser le long de guides (14) s'étendant dans la direction longitudinale du dit corps de conteneur (3).

6. Appareil selon la revendication 5, caractérisé en ce que ledit corps de conteneur (3) est ouvert sur son fond pour permettre le déplacement des dits éléments de conteneur (13).

7. Appareil selon l'une quelconque des revendications 1 à 6, caractérisé en ce que ledit corps de conteneur (3) est muni de pieds d'appui (12).

8. Appareil selon l'une quelconque des revendications 1 à 7, caractérisé en ce que les enroulements (4) sont placés de telle manière que la polarité nord du champ magnétique généré par les enroulements (4) est dirigée vers ledit plan de traitement (2).

9. Appareil selon l'une quelconque des revendications 1 à 8, caractérisé en ce que lesdits groupes (I,II,III) comprennent des enroulements côte-à-côte (4) disposés par paires de manière symétrique par rapport au plan de symétrie longitudinale du dit plan de traitement (2) et des enroulements simples (4) disposés de manière centrée par rapport au dit plan de symétrie longitudinale.

10. Appareil selon l'une quelconque des revendications 1 à 9, caractérisé en ce que lesdits groupes (I,II,III) comprennent des enroulements (4) disposés selon des densités différentes dans le corps de conteneur (3).
